# EUROPEAN PATENT APPLICATION

(11) **EP 2 570 138 A1**
(43) Date of publication of application: **20.03.2013**
(21) Application number: 12184263.7
(22) Date of filing: 13.09.2012
(51) Int. Cl.: A61L 2/26

(54) **Method and apparatus for handling non-sterilized objects in sterilized environment**

(30) Priority: 13.09.2011 US 201161533862 P
(71) Applicant: Sold, Adam D., Mill Valley, CA 94941 (US); Tzidon, Dekel, 45309 Hod Hasharon (IL)
(72) Inventor: Sold, Adam D., Mill Valley, CA 94941 (US); Tzidon, Dekel, 45309 Hod Hasharon (IL)
(74) Representative: Dawson, Elizabeth Ann

(57) **Abstract**

A device for operating a non sterilized tool within a sterilized room without tampering with the sterilization of the room is disclosed. The device comprises a first bag and a second bag. The second bag is located within the first bag and its opening edges are overlapping the opening edges of the first bag. The overlapping edges are adhered to each other to define an hermetic space between the first and the second bags and to maintain opened the opening of the second bag and that space is treated by means of one of sanitized, sterilized, disinfected or cleaned.

## Description

### FIELD OF THE INVENTION

The present invention relates to sanitized package. More particularly, the present invention relates to a dispensable sanitized package.

### BACKGROUND OF THE INVENTION

Today, consumer electronics, electronic devices and commonly available equipment have capabilities of high-end, specially-designed and sophisticated devices. In some cases the performance of such devices may be better than the equipment available for and allowed to be used in clean rooms and/or surgery rooms. One of the main obstacles in using such high-end, specially-designed and sophisticated devices inside clean rooms and/or surgery rooms is the requirement of sanitization of these devices. Surgery rooms, by definition should be sanitized including everything inside. There is a need for solution that will enable use of non-sanitized devices inside clean rooms and / or surgery rooms.

### SUMMARY OF THE INVENTION

A device for operating a non sterilized tool within a sterilized room without tampering with the sterilization of the room is disclosed, the device comprising a first bag, a second bag, said second bag is located within said first bag and having its opening edges overlapping the opening edges of said first bag, said overlapping edges are adhered to each other to define an hermetic space between said first and said second bags and to maintain opened the opening of said second bag, wherein said space is treated. Treated may be one of sanitized, sterilized, disinfected or cleaned.

sealing means disposed on the inner side of said second bag proximal to the edges of said bag, adapted to firmly attach one inner wall of said second bag to the wall against it

### BRIEF DESCRIPTION OF THE DRAWINGS

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings in which:

Fig. 1 illustrating a double bag with a sanitized enclosure in between two bags, in accordance with embodiments of the present invention;

Fig. 2 illustrating a device closed inside a bag with the sanitized surface after removing the outer bag, in accordance with embodiments of the present invention;

Fig. 3 depicts a double bag with sealing mechanism that may seal the common opening of a double bag when both sides are pressed against each other utilizing the omega shape of the closing edges, according to embodiments of the present invention;

Fig. 3A depicting single bag 120, after its outer bag has been removed, according to embodiments of the present invention;

Fig. 4 depicts a double bag with sealing mechanism with an integrated cutting edge that, when both sides are pressed against each other, may seal the common opening and cut the outer bag at the same time, according to embodiments of the present invention; and

Fig.5 depicts a double bag with a sealing mechanism that has adhesive surfaces or magnetic surfaces disposed on the inner side of the inner bag according to embodiments of the present invention; and

Fig. 6 is a flow diagram depicting method of inserting non-sterilized device into a sterilized room without tampering with the sterilization of the room, according to embodiments of the present invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION OF EMBODIMENTS OF THE PRESENT INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

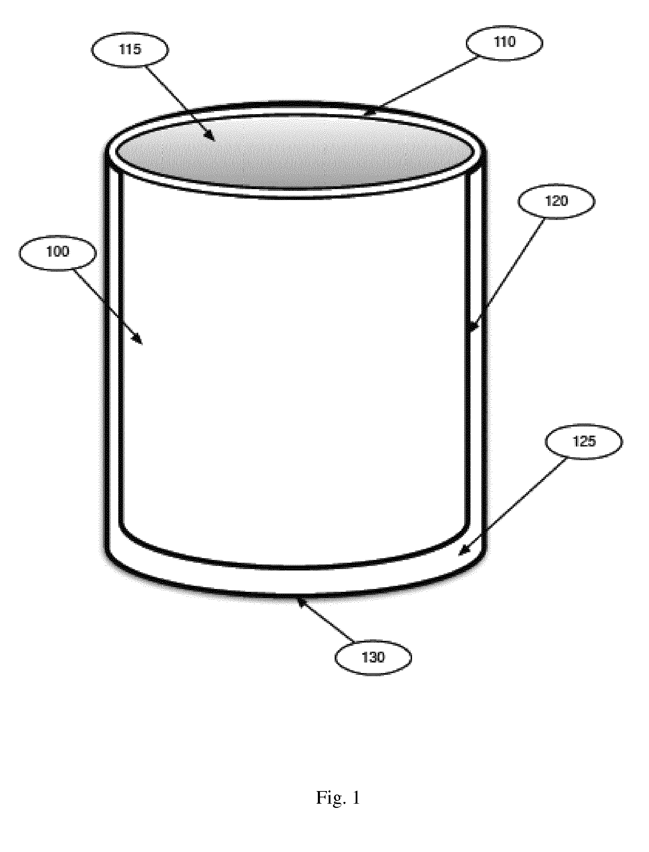

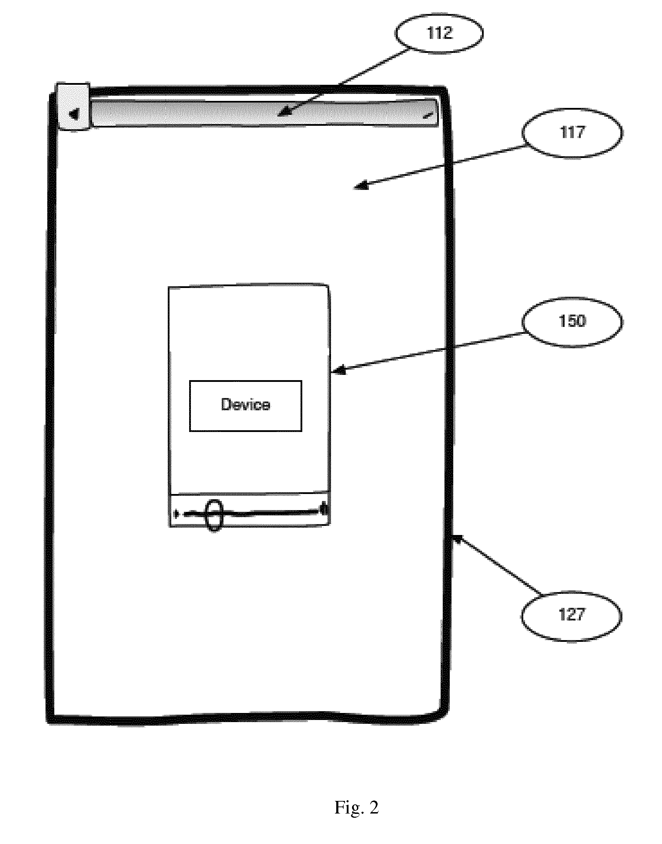

Reference is made now to **Fig. 1** illustrating double bag 100 with a sanitized enclosure in between the two bags, and to **Fig. 2****,** illustrating device 150 closed inside an internal bag 120 with the sanitized surface 127 facing outside, after removing outer bag 130, in accordance with embodiments of the present invention. Double bag package 100, in accordance with embodiments of the present invention, includes two bags, inner bag 120 and outer bag 130, one within the other, with common opening 110. The common opening 110 may be formed by firmly attaching or adhering the inner side of the edges of outer bag 130 to the outer side of the edges of inner bag 120 to create a hermetic sealing along the edges, hence the sanitized/treated inner bag 120 is protected by the outer bag 130. The terms 'adhere', 'connect', 'adjoin' and 'seal' may be used interchangeably and are used throughout the description of the present invention to describe firm attaching of one surface or edge of a sheet to another surface or edge, in a manner that provides hermetic connection along the adhered line. The space between the inner bag and the outer bag 125 is treated to a required level of sanitization and sealed hence inner space 125 defines a 'sanitized zone, between the outer face of the inner bag and the inner face of the outer bag. The inner side 115 of the inner bag may be open to the environment through the bags' common opening 110, thus defining a 'non-sanitized zone'. It will be noted that the terms "treat", "treated" and the like are used herein after to describe material / environment / space that was made to conform with sterilization / sanitation / disinfection / purification / antiseptically treated requirements.

Treated zone 125 may be empty (i.e. substantially vacuumed) or may be filled with treated air or any other suitable gas, liquid or powder.

Non-sanitized equipment that may used in sterilized environments according to e embodiments of the present invention may be any untreated equipment such as Computer, handheld computer, PDA, mobile phone, smartphone, flashlight, display, monitor, sensors, calculator, camera or any other device to be used inside controlled environment places. The double bag package 100 will allow such non-sanitized equipment 150 to be inserted into a controlled environment place, such as a surgery room when needed, without tampering with their level of sterilization, as is explained in details herein below. A non-sanitized device 150 may be inserted through common opening 110 into the inner space 115 defined by inner bag 120 and may be sealed inside this space using sealing mechanism 112. Before getting inside a clean room the outer bag 130 may be peeled off inner bag 120 leaving only the treated surface 127 (the outer face of inner bag 120) exposed to the environment.

Closing mechanism 112 may be designed to be treated and protected in sanitized zone 125 as well. Closing mechanism 112 may be designed for easy cutting and peeling the outer bag 130, for easier handling.

Additionally, closing mechanism 112 may be designed to be opened only once thus preventing the user from re-using a used double-bag 100 and forcing him to replace and use a new double bag package 100 on the next usage.

Optionally, the sanitized zone 125 may include an indicator (not shown) to indicate whether the 'sanitized zone' is sealed or broken or was tampered with.

Closing mechanism 112 of the common opening may be implemented in many ways, typically on the opening edges, such as adhesive, magnetic, zipper, rubber, welding and so on, to provide sufficient sealing after sanitization and allow easy opening/tearing off.

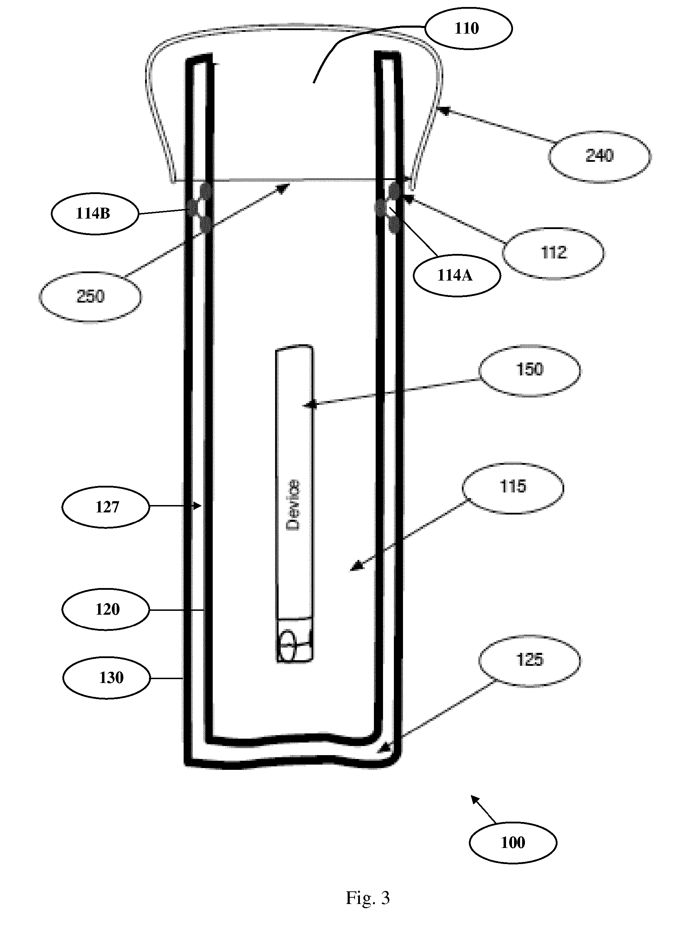

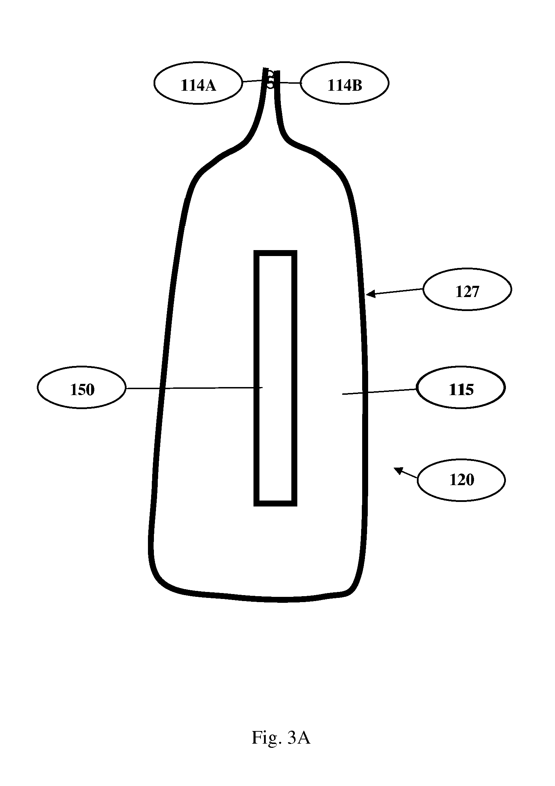

Figures 3, 4 and 5 illustrating schematic sectional-like side view of the double bag, with its common opening open, with various sealing and cutting mechanisms.

Fig. 3 depicts a double bag 100 with sealing mechanism 112 that may seal the common opening of a double bag when both sides are pressed against each according to embodiments of the present invention. Sealing mechanism 112 may seal the common opening 110 by snap attaching both sides of double bag 100 against each other utilizing the omega-like shaped profile 114A, 114B of the closing edges. It will be apparent that sealing profiles 114A, 114B may be formed in other shapes, as long as they provide firm attaching of one side of double bag 100 opening to the other. It should be noted that the sealing piece is located inside the sanitized zone 125 and thus may remain sanitized at all times. After the non-sanitized zone 115 is sealed, cutting tool 240 (shown in Fig. 3 in cross section view) may be slide along the sealing line close to and on the side of profiles 114A, 114B that is closer to the edges of bags 120, 130 and cut off these edges from inner bag while separating the inner bag from the outer bag with the cutting wire 250. Both the cutting tool 240 and the outer bag are non-sanitized materials and may be treated accordingly.

Reference is made now to Fig. 3A, depicting single bag 120, after its outer bag has been removed, according to embodiments of the present invention. After cutting tool 240 is operated and the edges of inner bag 120 and outer bag 130 are removed and outer bag 130 is peeled off inner bag 120, inner bag 120 remains stripped off outer bag 130 as seen in Fig. 3A. The opening of inner bag 120 remains firmly closed by means of snap attach profiles 114A, 114B. The outer face 127 of inner bag 120, which was sterilized beforehand, now faces outwardly and as a result bag 120 is sterilized on its outside and may be entered into a treated space, such as clean rooms or surgical rooms with device/tool 150 placed inside it. Accordingly device/tool 150 need not be sterilized and yet may be entered into clean room or surgical room without tampering with their sterilization. Tool/device 150 may be used and / or operated without been taken out of bag 120, for example used for measuring physical parameters (temperature, voice magnitude, etc.) or be used to take photos via the transparent wall of bag 120, and the like.

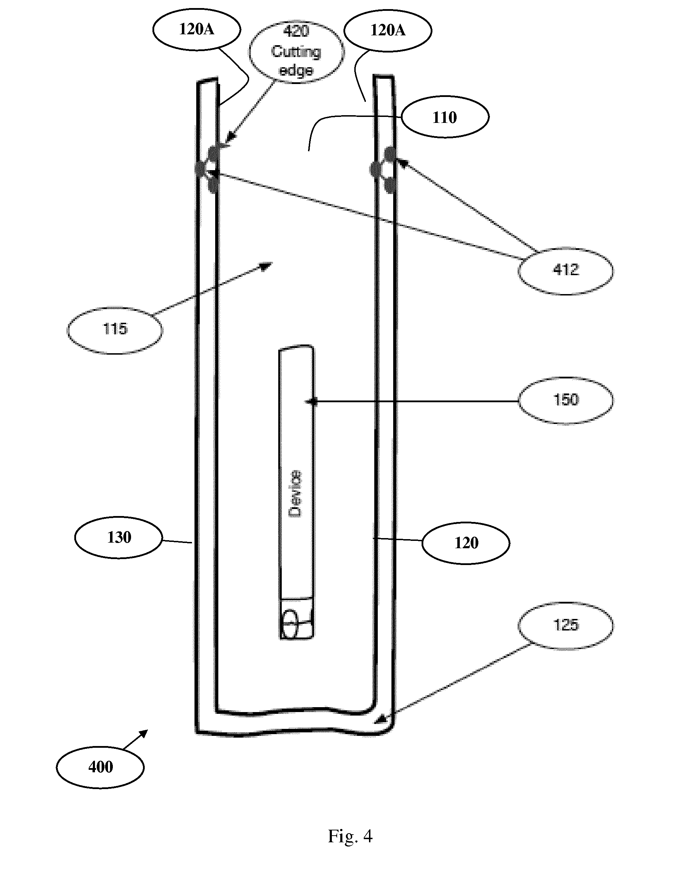

Fig. 4 depicts a double bag 400 with sealing mechanism 412 according to embodiments of the present invention. Double bag 400 may further comprise integrated cutting means at edges 420 proximal to sealing mechanism 412 that, when both sides are pressed against each other and firmly attached to each other may seal the common opening 110 and then the outer ends of inner bag 120 may be along the pre-prepared cutting lines to cut the outer bag 130 together with the outer ends 120A of inner bag 120 at the same time, according to embodiments of the present invention. Thus, the non-sanitized zone 115 is sealed, the outer bag 130 may be removed and thrown, leaving the sterilized outer face 127 of inner bag 120 facing out. Here also tool/device 150 is enclosed inside inner bag 120 and as such may be non-sterilized and yet may be entered inside clean or sterilized rooms.

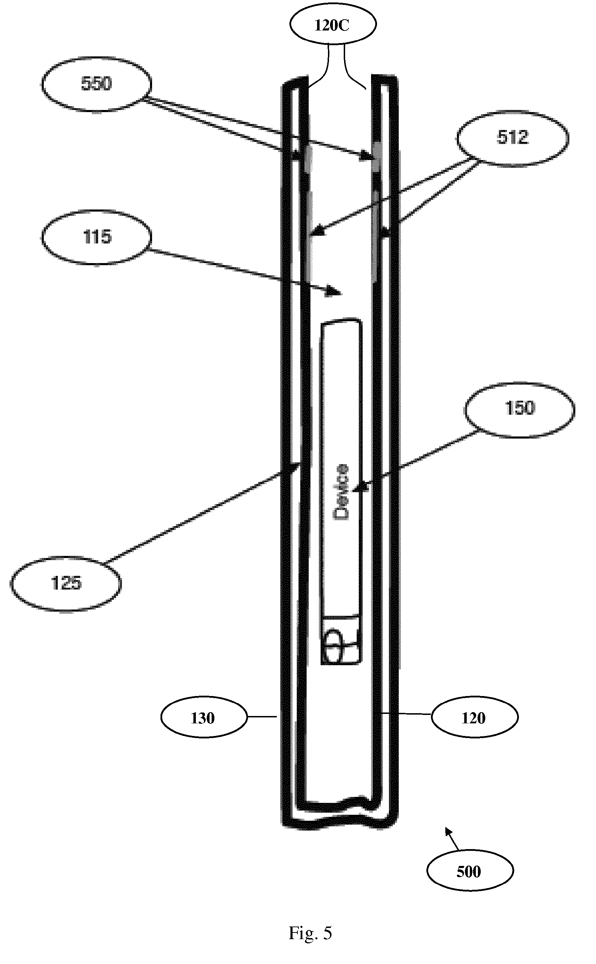

Fig.5 depicts double bag 500 with a sealing mechanism 512 that has adhesive surfaces or magnetic surfaces 512 disposed on the inner side of inner bag 120 close to its opening according to embodiments of the present invention. These adhesive or magnetic surfaces 512 may keep the adjacent sides of inner bag 120 close to its opening attached together and thus keeping the common opening sealed. In addition, a cutting mechanism 550 may be embedded in the common opening between surfaces 512 and the opening of bag 120; a pull-to-cut opening wire may be embedded in the inner bag wall and pulling the wire out will cut the outer edges 120C of bag 120 off together with out bag 130 which is connected to edges 120C. Alternatively a weakening line (such press made to lessen the width of the wall of inner bag 120 along the tear-off line) may be made in advance along a line between surfaces 512 and the edge of inner bag. Again, the 'non-sanitized zone' 115 remains sealed and the outer bag may be removed and thrown.

The inner bag may be made of materials such as PVC, Vinyl, Nylon, Polymer-based materials such as Polyxyene, Parylene, Polyethylene, Polypropylene, Polyurethane Polyethylene oxide, silicone containing polymer, Latex and other natural or synthetic rubber - elastic hydrocarbon polymer (polyisoprene).

Double-bag arrangements according to embodiments of the present invention are intended to allow use of non-sterilized equipment in sterilized locations, such as clean rooms, while kept inside the inner bag. Accordingly, the bags may be made of materials that meet the requirements of operation of that equipment from inside the inner bag. For example the bags may be made of transparent or opaque material or any combination thereof.

The inner bag may be made of a clear material for flashlight or camera use, soft or thin material for sensitive devices, sticky material for screens and displays, conductive/press-able/thin/sticky material for touch-screens and so on. Other material characteristics may be considered; Durability, tear resistance, gas and microorganisms impermeability and so on.

Additionally, advertisements may be printed on some designated areas of the inner and/or outer bags.

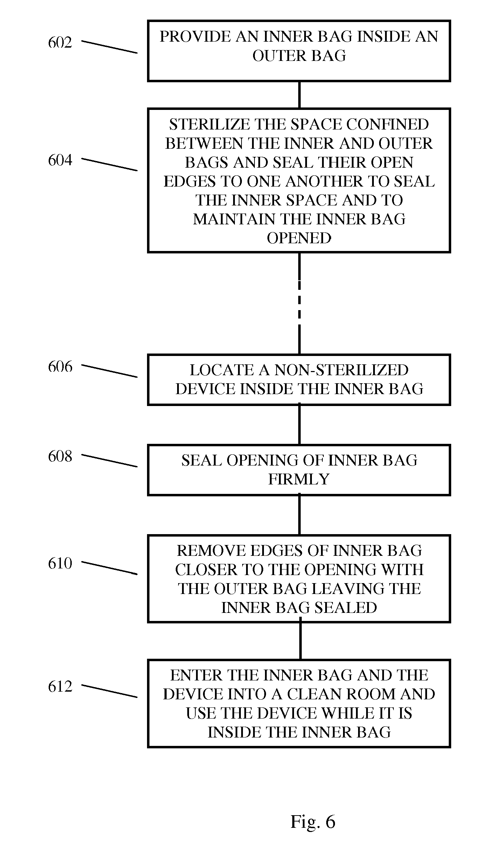

Reference is made now to Fig. 6, which is a flow diagram depicting method of inserting non-sterilized device into a sterilized room without tampering with the sterilization of the room, according to embodiments of the present invention. Two bags are provided the inner bag inside the outer bag, with their edges substantially overlapping (block 602). The space between the two bags is sterilized, sanitized, disinfected, purified or antiseptically treated and then the space between the bags is sealed by adhering or welding the overlapping edges of the inner bag to those of the outer bag, allowing the opening of the inner bag to remain opened (block 604). A non-sterilized (or non-treated) tool or device is inserted into the inner bag (block 606) and the opening of the inner bag is firmly closed along a line proximal to the edge of the bag (block 608). The wall of the inner bag is cut between the closing line and the edge of the bag and the edge of the inner bag together with the outer bag is removed off the inner bag, leaving the inner bag sealed and closed and exposing the outer face of the inner bag to the environment (block 610). The tool or device with the inner bag is entered into a sterilized room and the tool is operated within the bag without tampering with the sterilization of the room (block 612).

### Application use case:

The double bag package is structured to be handled between a person located in a clean room or sterile environment, like an operation room, and a person located outside that room.

In this case, an assistant, such as the preparation nurse, or an operator, may hold the bag and may insert the device 150 (whether sterilized or not) into the inner bag 120. The nurse will validate that the sterilization indicator, if present, indicating that the inner bag wasn't tampered with. The nurse may then seal the common opening of the bag with the sealing mechanism, either by stripping the adhesive stripe or by zipping it close with the sealing and cutting zipper edge. As a result, the device 150 will be sealed in the inner bag that is now separated from the outer bag 130, but is still inside it.

Next, the preparation room nurse or the operator may hand the bag with the sealed device in it to a second person, such as a nurse or an operator located in the clean or sterile room. After the second nurse holds on to the sterile inner bag, the preparation room nurse may strip the non-sterile outer bag from the sterile inner bag of the pouch. The sterile indicator, if present in this example, may indicate that the outer side of the inner bag has been exposed to the air and is no longer sealed in a long term sterile environment.

At this point, the preparation room (first) nurse is left with the entire outer, non-sterile layer, while the inner sterile bag, with the device sealed in it, is left in the hands of the second nurse in the operation theatre.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the true spirit of the invention.

## Claims

1. A device for operating a non sterilized tool within a sterilized room without tampering with the sterilization of the room, the device comprising:
a first bag;
a second bag, said second bag is located within said first bag, having its opening edges overlapping the opening edges of said first bag, said overlapping edges are adhered to each other to define an hermetic space between said first and said second bags said space is treated, and to maintain opened the opening of said second bag;
sealing means disposed on the inner side of said second bag proximal to the edges of said bag, adapted to firmly attach one inner wall of said second bag to the wall against it

2. The device of claim 1 further comprising cutting means adapted to cut said second bag around its sides between said sealing means and said edges.

3. The device of claim 1 wherein said treated space is empty.

4. The device of claim 1 wherein said treated space is occupied by a treated material selected from a list comprising gas, liquid, and powder.

5. The device of claim 1, wherein said sealing means is located inside said treated space and selected from a list including snap-attach profile, adhesive and magnetic surfaces.

6. The device if claim 1 wherein said treated is one of sanitized, sterilized, disinfected or cleaned.

7. The device of claim 1 wherein said second bag is made of material characterized to be at least one of transparent, electric conductive and enabling to operate a touch screen through it.

8. The device of claim 1 further comprising an indicator to indicate the status of said treated space.

9. A method for enabling insertion a non-sterilized tool into a sterilized room without tampering with its sterilization comprising:
providing a first and a second bags, said second bag is located within said said first bag with its opening edges substantially overlapping the opening edges of said first bag;
treating the space defined between said first and said second bag;
adhering said overlapping edges of said first bag to those of said second bag, to seal the treated space defined between said first and said second bags and maintain the opening of said second bag opened;

10. The method of claim 9 further comprising inserting a non-sterilized tool into said second bag.

11. The method of claim 10 further comprising sealing the opening of said second bag along a sealing line.

12. The method of claim 11 further comprising cutting the edge of said second bag along a line located between said sealing line and the edges of said second bag.

13. The method of claim 12 further comprising peeling said first bag off said second bag and exposing the outer face of said second bag to the ambient, said outer face is sterilized.

14. The method of claim 13 further comprising inserting said second bag inside the sterilized room.

15. The method of claim 14 further comprising operating said tool inside said second bag.
